(19) Europäisches Patentamt — European Patent Office — Office européen des brevets

(11) **EP 3 697 444 B1**

(12) **EUROPEAN PATENT SPECIFICATION**

(45) Date of publication and mention
of the grant of the patent:
**09.03.2022 Bulletin 2022/10**

(21) Application number: **18803474.8**

(22) Date of filing: **17.10.2018**

(51) International Patent Classification (IPC):
*A61K 47/20* (2006.01)        *A61K 9/08* (2006.01)
*A61K 31/728* (2006.01)       *A61P 43/00* (2006.01)
*A61K 45/06* (2006.01)        *A61K 31/10* (2006.01)

(52) Cooperative Patent Classification (CPC):
(C-Sets available)
**A61K 9/08; A61K 31/10; A61K 31/728;
A61K 47/20; A61P 43/00;** A61K 9/0014;
A61K 9/0019                              (Cont.)

(86) International application number:
**PCT/IB2018/058051**

(87) International publication number:
**WO 2019/077521 (25.04.2019 Gazette 2019/17)**

(54) **LIQUID COMPOSITION FOR USE AND AS AN ANTI-INFLAMMATORY**

FLÜSSIGE ZUSAMMENSETZUNG ZUR VERWENDUNG ALS ENTZÜNDUNGSHEMMER

COMPOSITION LIQUIDE DESTINÉE À ÊTRE UTILISÉE COMME ANTI-INFLAMMATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **17.10.2017 IT 201700117361**

(43) Date of publication of application:
**26.08.2020 Bulletin 2020/35**

(73) Proprietor: **Alfakjn S.r.l.**
**27026 Garlasco (PV) (IT)**

(72) Inventor: **FERRARI, Alessio**
**27026 Garlasco (PV) (IT)**

(74) Representative: **Hoffmann Eitle**
**Hoffmann Eitle S.R.L.**
**Piazza Sigmund Freud 1**
**20154 Milano (IT)**

(56) References cited:
**EP-A1- 1 444 984          EP-B1- 1 444 984
WO-A1-94/05293             WO-A1-2017/025903
US-A1- 2015 094 279**

(52) Cooperative Patent Classification (CPC): (Cont.)

    C-Sets
    **A61K 31/10, A61K 2300/00;**
    **A61K 31/728, A61K 2300/00**

**Description**

The present invention relates to a composition for the treatment and/or prevention of disorders and/or pathologies linked to oxidative and/or inflammatory states as presently claimed.

The use of hyaluronic acid and the salts and derivates thereof for the treatment and prevention of disorders including arthrosis, by intra-articular infiltration, otological disorders (regeneration of perforated tympanic membranes), and inflammations and ulcerous lesions of the skin and mucosa, for example of the mouth, such as *aphthae* and stomatitis, is well known.

Hyaluronic acid is moreover widely used in dermatology and for cosmetic purposes as a main component of various topical products such as facial fillers, gels, sprays and mouthwashes.

*In vivo,* hyaluronic acid is metabolised by specific enzymes belonging to the hyaluronidase family, which provoke its degradation.

One of the main disadvantages of the use of hyaluronic acid *in vivo* is thus its relatively rapid degradation by hyaluronidases. It follows that in order to obtain significant, lasting effects, the administration of hyaluronic acid must be repeated over time.

WO 2017/025903 A1 discloses an injectable liquid composition comprising an effective amount of a mixture which comprises or, alternatively, consists of hyaluronic acid (and/or a pharmaceutically acceptable salt thereof) and methylsulfonylmethane, and a physiologically acceptable carrier; said composition being for use in the intra-articular infiltration therapy.

EP 1 444 984 A1 discloses compositions comprising an association of hyaluronic acid, *Melaleuca alternifolia* extracts and methyl-sulphonylmethane (MSM) suitable for topical administration on human and animal mucous membranes, in particular for the prevention and/or treatment of inflammatory processes of mucosae.

WO 94/05293 A1 discloses compositions comprising methylsulphonylmethane and at least one of oxypurinol and allopurinol, and their use in formulations and methods of treatment and prophylaxis of one or more of skin conditions, diseases and injuries.

US 2015/094279 A1 discloses a pharmaceutical composition for inhibiting inflammation comprising hyaluronic acid, a vitamin and a pharmaceutically acceptable carrier.

There is still a felt need to be able to have a formulation that enables hyaluronic acid to perform a prolonged action that is fundamentally free of side effects.

One object of the present invention is to provide a hyaluronic acid formulation that is stable, easy to use, fundamentally free of side effects and enables a prolonged action of hyaluronic acid to be obtained.

As a solution to said need, the present invention provides a composition for use according to the appended claims.

The present invention relates to a liquid composition comprising (i) an effective amount of a mixture that comprises or, alternatively, consists of at least (a) hyaluronic acid, or a salt thereof, and (b) methylsulfonylmethane (MSM), wherein (a) and (b) are in a ratio by weight between 1:2 and 1:4, and (ii) a physiologically acceptable carrier; wherein said composition is for use in a method for the preventive or therapeutic treatment of at least one disorder or pathology deriving from an oxidative and/or inflammatory state, wherein said disorder or pathology is at least one selected from: vulvovaginal atrophy, vaginal dryness, abacterial cystitis, prostatitis, rhinitis, sinusitis, rhinorrhoea, allergic rhinitis, haemorrhoids, anal fissures, rhagades and lesions of the rectal canal; wherein said composition is administered to a subject in a state of need and wherein said composition is administered to said subject topically.

The present description relates to the composition as defined above for use as a medication.

The present description further relates to a pharmaceutical composition or medical device (a composition for a medical device) comprising the liquid composition as defined above. Preferred embodiments of the present invention will emerge clearly from the detailed description that follows and are specified in the appended claims.

Figure 1a is a correlogram of the comparative sample A diluted in $H_2O$ at two different concentrations.

Figure 1b shows the distribution of hydrodynamic radii of the comparative sample A diluted in $H_2O$ at two different concentrations.

Figure 2 is a correlogram of A diluted in $NaNO_3$ at three different concentrations.

Figures 3a and 3b show, respectively, the results relating to A and B diluted in $NaNO_3$ 0.1M.

Figure 4 is a $^1$H NMR of the comparative sample A, Figure 5 is a $^1$H NMR of the comparative sample S3410 and Figure 6 is a $^1$H NMR of the comparative sample B.

Figure 7 is an overlay of the three $^1$H NMR spectra of the three figures 4-6 and Figure 8 is the expansion of the spectral region around 3 ppm in Figure 7.

Figure 9 shows the hydrolysis kinetics of samples A and B.

Figure 10 shows the reduction in IL-1$\beta$ at the different concentrations of the tested samples.

Figure 11 shows the reduction in IL-6 at the different concentrations of the tested samples.

EP 3 697 444 B1

Figure 12 shows the reduction in TNF-$\alpha$ at the different concentrations of the tested samples.

Within the scope of the present invention, "treatment" of a pathology or disorder means a therapy aimed at restoring the health conditions of a subject, maintaining the existing conditions and/or preventing a worsening of said health conditions.

Within the scope of the present invention, "prevention" of a pathology or disorder means therapy aimed at avoiding the onset of such a pathology or disorder in a subject, also, but not only, as a complication or effect of a pre-existing pathological condition or disorder.

Unless specified otherwise, within the scope of the present invention the percentages and amounts of a component in a mixture are intended to refer to the weight of that component relative to the total weight of the mixture.

Unless specified otherwise, within the scope of the present invention, in relation to intervals of numerical values for a certain feature, the indication "from X to Y" comprises the extremes, i.e. X and Y, as well as all the possible intermediate numerical values.

In the context of the present invention, the term "composition(s)" is meant to include a pharmaceutical composition or a composition for a medical device.

In one aspect, the present invention provides a liquid composition comprising: (i) an effective amount of a mixture that comprises or, alternatively, consists of at least (a) hyaluronic acid, or a salt thereof, and (b) methylsulfonylmethane (MSM), in cui (a) and (b) are in a ratio by weight between 1:2 and 1:4, and (ii) a physiologically acceptable carrier.

Following extensive trials, the inventors have developed a liquid composition comprising hyaluronic acid and methylsulfonylmethane (MSM) which, in specific weight proportions, can form a non-covalent complex, or compound through a strong electrostatic bond. The formation of said compound makes it possible to obtain a more prolonged action of hyaluronic acid compared to the currently available formulations.

Without wishing to be limited by theory, it is possible to affirm that the presence of said strong electrostatic bond brings about the formation of a complex with dimensions and a molecular weight exceeding those of hyaluronic acid on its own. An increase has also been observed in the viscosity of the composition according to the invention compared to analogous solutions containing hyaluronic acid alone or hyaluronic acid and MSM in proportions outside the ranges indicated above.

Following administration of the composition according to the invention, it has been observed that, probably as a result of the formation of said non-covalent bond, the hyaluronic acid is metabolised more slowly by hyaluronidase compared to what is observed for analogous solutions containing hyaluronic acid alone or hyaluronic acid and MSM in proportions outside the ranges indicated above. This results in a prolonged action of hyaluronic acid, with the final advantage that, compared to known compositions, a smaller number of administrations of preparations containing hyaluronic acid are necessary to obtain comparable physiological effects.

The reduction in the number of administrations makes it possible to minimise the risk of side effects and improve the compliance of subjects who undergo the treatment.

In the composition according to the present invention, the ratio by weight between (a) and (b) is preferably between 1:2.5 and 1:3.5; said ratio is preferably 1:3.

In the composition according to the present invention, the carrier is in liquid form (liquid carrier) and is pharmacologically, i.e. physiologically, acceptable as it contains, for example, a liquid vehicle (water for injectable preparations), formulation additives, diluents and pharmaceutically acceptable excipients. Said carrier in liquid form preferably comprises a non-pyrogenic sodium chloride and/or disodium hydrogen phosphate anhydrous -$Na_2HPO_4$, and/or sodium dihydrogen phosphate dihydrate -$NaH_2PO_4 \times 2H_2O$, and/or water for injectable preparations.

The hyaluronic acid, or a pharmaceutically acceptable salt thereof, is a linear, unbranched hyaluronic acid, preferably of biofermentative origin.

The hyaluronic acid, or a pharmaceutically acceptable salt thereof, can have a weight average molecular weight ($M_w$) comprised from 700 KDa to 1400 KDa, preferably 800-1000 KDa. It has been found that this range of molecular weight ensures an ideal affinity for the hyaluronic acid receptors, an ideal duration of prolonged action and a better stimulation of the biosynthesis of hyaluronic acid.

In the composition according to the invention, the hyaluronic acid, or a salt thereof, preferably (a) has a weight average molecular weight of 700 KDa to 1400 KDa, preferably 800-1200 KDa.

A pharmaceutically acceptable salt of hyaluronic acid is sodium hyaluronate.

The composition of the present invention preferably contains hyaluronic acid, and/or a pharmaceutically acceptable salt thereof, in an amount by weight comprised from 0.5% to 6%, more preferably 1% to 3.5% or 1.5% to 3.2% or 4.5% to 5.5%, relative to the total weight of the composition.

The composition of the present invention preferably contains methylsulfonylmethane (MSM, CAS number 67-71-0) in an amount by weight comprised from 1% to 12%, more preferably 2% to 7% or 3% to 6.8% or 9% to 11%, relative to the total weight of the composition.

It has been found that a composition comprising an amount of MSM comprised in the ranges indicated above,

4

moreover, enables effectiveness in therapy to be maximised, for example in parenteral infiltration therapy, and side effects to be minimised, in particular the burning sensation at the moment of the injection.

The composition according to the present invention can be for therapeutic use in human subjects or for veterinary use, for example, but without limitation, in pets such as dogs or cats, or in other mammals. The composition according to the present invention is preferably for use in humans.

The liquid composition for use according to the present invention, comprising (i) an effective amount of a mixture that comprises or, alternatively, consists of at least (a) hyaluronic acid, or a salt thereof, and (b) methylsulfonylmethane (MSM), wherein (a) and (b) are in a ratio by weight between 1:2 and 1:4, and (ii) a physiologically acceptable carrier, is formulated in solid, liquid or semi-solid (i.e. gel) form in order to be administered to subjects in a state of need through an appropriate route known to the person skilled in the art. Said formulations (solid, liquid or semi-solid) of the aforesaid liquid composition for use of the present invention correspond to pharmaceutical compositions or compositions per medical devices described in the present invention.

The administration of the composition to the subject takes place topically, for example by application of an ointment, cream, gel, unguent, spray, suspension or solution containing the composition according to the invention directly on a part of the body of a subject to be treated. More preferably, the composition according to the present invention is in the form of a solution. Preferred but non-limiting examples of formulations (in the form of solutions) for topical application are vaginal douches, nasal sprays, nasal washes, vesicle solutions and micro-enemas. The composition according to the present invention is preferably formulated in the form of a nasal spray or nasal washes.

Advantageously, when the liquid composition for use of the invention, comprising (i) an effective amount of a mixture that comprises or, alternatively, consists of at least (a) hyaluronic acid, or a salt thereof, and (b) methylsulfonylmethane (MSM), wherein (a) and (b) are in a ratio by weight between 1:2 and 1:4, and (ii) a physiologically acceptable carrier, is administered to said subject topically, said composition is formulated in liquid form.

Alternatively, when the liquid composition of the invention, comprising (i) an effective amount of a mixture that comprises or, alternatively, consists of at least (a) hyaluronic acid, or a salt thereof, and (b) methylsulfonylmethane (MSM), wherein (a) and (b) are in a ratio by weight between 1:2 and 1:4, and (ii) a physiologically acceptable carrier, is administered to said subject topically, said composition is formulated in gel form. The gel is an elastic biphasic colloidal material or consists of a liquid that is dispersed and incorporated in the solid phase. Preferred but non-limiting examples of formulations in gel form for topical application are a rectal gel, vaginal gel, buccal gel, nasal gel, ocular or periocular gel, topical or dermal gel. More preferably, the composition according to the present invention is formulated in the form of a gel for rectal application.

It remains understood that the administration according to the invention can take place simultaneously, for example in a single formulation, or in rapid sequence, for example by means of two or more formulations taken by the subject in any order, in a sequence closely spaced over time (e.g. within 1 to 5 minutes) in two distinct compositions.

The composition for use according to the present invention may comprise, in addition to (a), (b) and a pharmaceutically acceptable carrier, at least one inert ingredient, such as at least one excipient among those commonly used and known to the person skilled in the art.

"Inert ingredient" means any substance, or combination of substances, auxiliary to the production of a pharmaceutical, dietary or nutraceutical form, which is to be found in the finished product and is not the active ingredient, although it can modify the stability, release or other characteristics thereof.

Non-limiting examples of such ingredients, as known to the person skilled in the art of formulations in the pharmaceutical sector, are diluent, colouring, surfactant, antioxidant, sweetening, flavouring, viscosity-enhancing, emulsifying and humectant excipients, preservatives, vitamins, lactic acid bacteria, plant extracts, chelating agents, sodium chloride, methyl and ethyl parabens, sodium benzoate, phenoxy ethanol, potassium sorbate, sodium phosphate dibasic, sodium phosphate monobasic, sodium carbonate, sodium citrate, sodium dehydroacetate, *glycyrrhetic* acid, cellulose derivatives, polysorbates, glycerine, glycerol, ethyl alcohol and the like.

In a preferred embodiment, the composition for use according to the present invention comprises, in addition to (a), (b) and a pharmaceutically acceptable carrier as defined above, at least one further active ingredient of natural or synthetic origin. Non-limiting examples of said active ingredients are lipo- or watersoluble vitamins (A, C, E etc..), live or tyndallised lactic acid bacteria, plant extracts, phospholipids (also as micelles or liposomes), anti-inflammatories, antibiotics, vasoconstrictors and saliva substitutes.

The composition according to the present invention is for use as a medication.

The composition according to the invention is for use in a method for the preventive or therapeutic treatment of present claimed disorder or pathology deriving from an oxidative and/or inflammatory state, wherein said treatment comprises the administration of said composition to a subject.

The composition according to the present invention is for use in a method for the treatment of at least one disorder or pathology among vulvovaginal atrophy, vaginal dryness, abacterial cystitis, prostatitis, rhinitis, sinusitis, rhinorrhoea, allergic rhinitis, haemorrhoids, anal fissures, rhagades and lesions of the rectal canal. The liquid composition according to the present invention for use in the aforesaid treatment methods is formulated in liquid form, such as, for example, in the form of a solution, or in semisolid form, such as, for example, in the form of a gel.

The subject matter of the present description further relates to a method for the preventive and/or curative treatment of at least one disorder or pathology deriving from or linked to an oxidative and/or inflammatory state, wherein said method of treatment comprises the administration of the composition of the invention, comprising (i) an effective amount of a mixture that comprises or, alternatively, consists of at least (a) hyaluronic acid, or a salt thereof, and (b) methylsulfonylmethane (MSM), wherein (a) and (b) are in a ratio by weight between 1:2 and 1:4, and (ii) a physiologically acceptable carrier, to a subject having need. Said treatment method preferably comprises topical rectal administration of the composition of the invention to said subject; in such a case, the composition of the invention is preferably formulated in gel form.

Alternatively, said treatment method comprises topical nasal administration of the composition of the invention to said subject; in such a case, the composition of the invention is preferably formulated in the form of a liquid suitable for nasal applications (nasal liquid or spray).

In one aspect, the present description relates to a pharmaceutical composition or medical device (composition for a medical device) comprising the liquid composition comprising at least (a), (b) and a pharmaceutically acceptable carrier as described above.

In the context of the present invention, the term "medical device" is used with the meaning according to Italian Legislative Decree no. 46 of 24 February 1997 and Directive 93/42/EEC of 14 June 1993, i.e. it indicates a substance or another product, whether used alone or in combination, intended by the manufacturer to be used for human beings for the purpose of diagnosis, prevention, monitoring, treatment or alleviation of disease, and which does not achieve its principal intended action in or on the human body for which it is intended by pharmacological, immunological or metabolic means, but which may be assisted in its function by such means.

The following experimental part provides examples of practical embodiments of the invention.

EXPERIMENTAL PART A

The aim of the study is the physicochemical characterisation of a sample of hyaluronic acid (HA) dissolved in an aqueous solution with or without methylsulfonylmethane (MSM). Since it is hypothesised that a non-covalent interaction occurs between the polysaccharide and the organic molecule, generating complexes of an ionic nature, use was made of techniques capable of determining the molecular weight of the polymer, the hydrodynamic radius and the mobility of the complex in a solution.

**Materials and Methods**

The samples undergoing the analysis were coded as shown in Table 1:

**Table 1: List of samples**

| Sample | Description |
| --- | --- |
| HA 1.6% + MSM 5%, (sample B according to the invention, G13364) | Product in a syringe |
| Condronil IAL HA 1.6% (comparative sample A, G13365) | Product in a syringe |
| Methylsulfonylmethane (MSM), lot 17010616 | Product in powder form |

**HP-SEC/TDA analysis**

The distribution of molecular weights is determined with the instruments and under the chromatographic conditions stated below:

*Instrument*: OmniSEC System, Malvern Instruments.
*Columns*: two GMPWXL columns, 7.8 mm ID x 30 cm, Tosoh Bioscience.
*Temperature*: 40°C.
*Mobile Phase*: $NaNO_3$ 0.1 M, $NaN_3$ 0.05%.
*Flow*: 0.6 ml/min.
*Detector*. Refractive index, viscometer, *Right Angle and Low Angle Light Scattering.*
*Injection volume*: 100 μl.

*Detector calibration*

The instrument was calibrated with a Pullulan standard, of which the weight average molecular weight, intrinsic viscosity and concentration are certified (PolyCAL Pullulan-57K-Std TDS3031, *Malvern Instruments).*

Sample Preparation

The solutions were diluted with the mobile phase so as to obtain an HA concentration of about 0.4 mg/ml.

Processing

The data were processed with dedicated software (OmniSEC, version 4.6); a chromatogram between 0 and 36 ml was acquired, the eluted peak between 8.5 and 15 ml was integrated and a dn/dc value equal to 0.155, previously determined in the Institute's laboratory, was imposed.

**Dynamic Light Scattering analysis**

The values of the hydrodynamic radius (PCS experiment) and zeta potential were determined with the instruments and under the chromatographic conditions stated below:

Instruments

A DLS Zetasizer Nano ZS instrument *(Malvern Instruments)* was used.
The acquisition parameters for the experiments are the following:

∘ Parameters for PCS:

- Material: Protein
- Dispersant: water
- Temperature: 25°C
- Time to reach system equilibrium: 30 sec
- Cell: Disposable sizing cuvette ZEN0040 (Malvern Instruments)
- Number of runs: 10
- Duration of a run: 10 sec
- Number of experiments: 3
- Delay between experiments: 5 sec
- Automatic attenuation
- Data processing: General purpose

∘ Parameters for Zeta Potential:

- Material: Protein
- Dispersant: water
- Model: Smoluchowsky F(Ka) = 1.50
- Temperature: 25°C
- Time to reach system equilibrium: 30 sec
- Cell: disposable folded capillary zeta cell (DTS10710)
- Number of runs: 10
- Number of experiments: 6
- Delay between experiments:5 sec
- Automatic attenuation selection: Yes
- Automatic attenuation
- Data processing: Single-mode

Sample preparation

The solutions were diluted with the mobile phase used for the HP-SEC/TDA analysis in order to obtain an HA concentration of about 1.6 mg/ml, 0.32 mg/ml and 0.16 mg/ml.

**NMR analysis**

A *Bruker Avance* III HD spectrometer operating at 500 MHz and equipped with a homo- and heteronuclear cryogenic probe (TCI *Cryoprobe)* was used for the NMR analyses.

$^1$H-NMR instrument parameters:

- pulse program: zgcppr
- number of scans: 16
- D1: 12 seconds
- TD: 32K
- spectral window: 18 ppm
- temperature: 303K

Sample preparation

The hyaluronic acid-based samples already dissolved in water were diluted using 99%D deuterated water and the solvent was exchanged with deuterated water at least twice. The samples that were analysed had a concentration of about 8 mg of HA in 600 $\mu$l of $D_2O$. The powder sample of MSM was directly dissolved in $D_2O$ at a concentration of 1 mg in 600 $\mu$l.

**Results**

**High Performance-Size Exclusion Chromatography-Triple Detector Array analysis**

With the aim of determining the distribution of molecular weights, the samples were subjected to size-exclusion chromatographic analysis using a combination of four detectors (light scattering at 90° and 8°, refractive index and viscometer). A first reprocessing of the chromatograms enables the distribution of molecular weights and thus Mn (number molecular weight), Mw (weight molecular weight) and Mw/Mn (polydispersity) to be determined (Reference: Compendium of Polymer Terminology and Nomenclature IUPAC Recommendations 2008- "Purple book" RSC Publishing). Using the viscosimeter, the value of [$\eta$] (intrinsic viscosity) is derived for each molecular weight value. The latter data enable a determination of the Mark-Houwink parameters (a and K), which, appropriately processed, together with the light scattering data, provide information regarding both the macromolecular size (radius of gyration Rg) and any branching of the macro molecule.

In order to process the chromatographic data by means of OminSEC software, it is necessary to know the exact concentration of the sample by imposing the dn/dc value of the same dissolved in a solution. All samples were injected in duplicate, according to the procedures set forth above. Shown below is a table summarising the results obtained (Table 2) as the average of two injections; the table lists the number average molecular weight (Mn) and weight average molecular weight (Mw), both expressed in Daltons, polydispersity (Pd), intrinsic viscosity [$\eta$], expressed in dl/g, hydrodynamic radius (Rh), expressed in nm, and percentage recovery, which is calculated as the percentage ratio between the concentration derived from the area of the refractive index resulting from the imposition of the correct dn/dc value and the concentration prepared.

The chromatograms of the injections carried out are also shown (see appended figure).

**Table 2: HP-SEC/TDA results**

| Sample | Mw (Da) | Mn (Da) | Mw/Mn | $\eta$ (dl/g) | Rh (nm) | % recovery |
|---|---|---|---|---|---|---|
| **A** | 749100 | 532400 | 1.41 | 12.72 | 51.6 | 111 |
| **B** | 860900 | 552900 | 1.56 | 14.11 | 56.0 | 102 |

The values of the weight and number average molecular weight increase for sample B versus sample A, as do the values of the hydrodynamic radius and intrinsic viscosity. This could be correlated to the presence of MSM, which increases the molecular weight of the polymer, to which in this case it would be bound by interactions sufficiently strong so as not to cease during the chromatography.

**Dynamic Light Scattering (PCS) and Zeta Potential (Zp) analysis**

The hydrodynamic radius of the products was evaluated by dynamic light scattering (DLS). The technique measures the speed at which particles are moving by Brownian motion in a solution, be it homogeneous or colloidal: larger particles move more slowly in it than smaller particles. In particular, the technique measures the fluctuations over time in the light scattering intensity and analyses the time/scattering parameters by means of an autocorrelation function G(t) (Reference: international standard ISO 13321:1996 and subsequent versions and manuals of the instrument manufacturer Malvern, for example : http://149.171.168.221/partcat/wp-content/uploads/Malvern-Zetasizer-LS.pdf).

This function decays, in an interval of time t, according to an exponential law:

$$G = A * e\text{-}2\ \Gamma\ T\text{+}1$$

(where A is an amplitude constant, t is the time, $\Gamma$ is the decay constant).

In turn, $\Gamma$ depends on the diffusion coefficient Dt and the parameter q:

$$\Gamma = Dt * q2$$

$$Dt = Kt\ /\ 6\ \pi\ \eta\ Rh$$

$$q = (4\ \pi\ (dn/dc)\ /\ \lambda)* \sin\ (\Phi)$$

Where:

K = the Boltzmann constant
t = temperature
$\eta$ = solvent viscosity
Rh = hydrodynamic radius
n = solvent refractive index
$\lambda$ = laser wavelength
$\Phi$ = scattering angle

The analysis times are very short (from 1 to 5 sec); it is thus possible to repeat the measurement a number of times in order to reduce the variability of individual measurements; the software is capable of calculating an average decay curve from the set of these curves.

A first result that is obtained by processing the experimental data via dedicated software is the distribution of the hydrodynamic radii of the various species present in the solution. By means of subsequent mathematic reprocessing it is possible to obtain the distribution of the average hydrodynamic radii correlatable to the molecular weight.

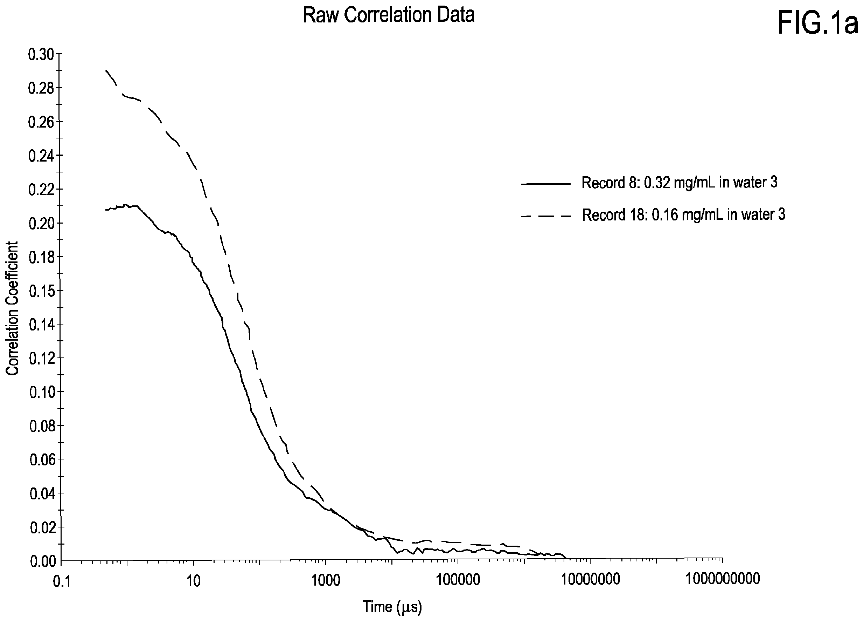

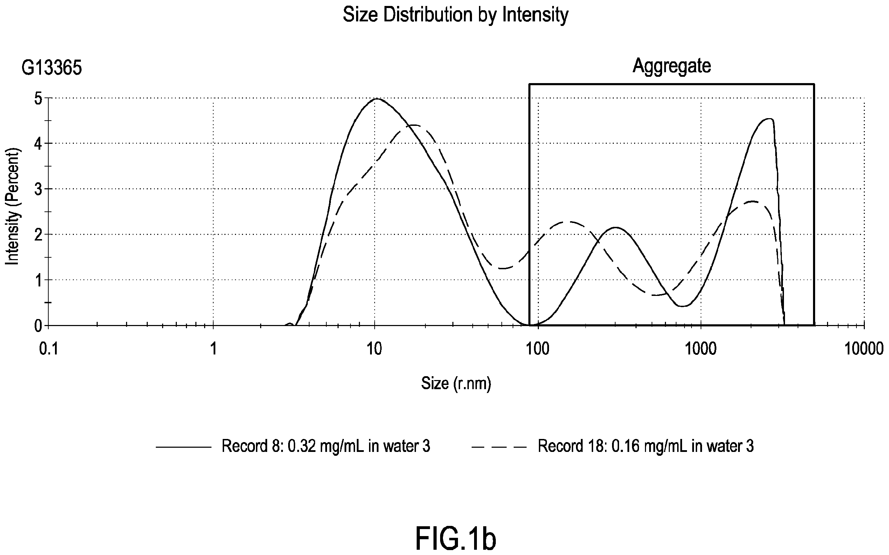

The Z-average value is an average particle size, which as such also takes into account the presence of aggregate structures or shorter chains. However, given the extremely polydispersed nature of the sample and the tendency of charged polymers to aggregate, this parameter is judged to be the most significant for comparing samples, as opposed to an analysis of the radii of the different species present. Figure 1 shows the analysis of HA (A) and HA with MSM (B) at different concentrations.

**Table 3: PCS results with dilution in water**

| Sample | [HA] (mg/ml) | [MSM] (mg/ml) | Z-Average (nm) | Standard deviation | Dilution |
|---|---|---|---|---|---|
| A | 0.32 | 0 | 24 | 3.1 | $H_2O$ |
|  | 0.16 | 0 | 30 | 7.7 |  |
| B | 0.32 | 1.0 | 22 | 1.92 | $H_2O$ |
|  | 0.16 | 0.5 | 48 | 16.6 |  |

The correlation functions obtained from these first experiments showed a great deal of noise due to the presence of aggregate structures in significant amounts. Figure 1a shows, as an example, the cumulative curves obtained for sample A and the size distribution obtained from the mathematic analysis of the curve (Figure 1b).

The presence of aggregate chains obviously influences the Z-average value determined. In order to reduce this phenomenon, it was decided to dilute in 0.1 M $NaNO_3$, salt used in the mobile phase of the GPC analyses. In the presence of salts, charged polysaccharides normally tend to considerably reduce the electrostatic interactions responsible for aggregation phenomena. Using a saline solution for dilution, it was also deemed appropriate to analyse a more concentrated solution (1.6 mg/ml of HA).

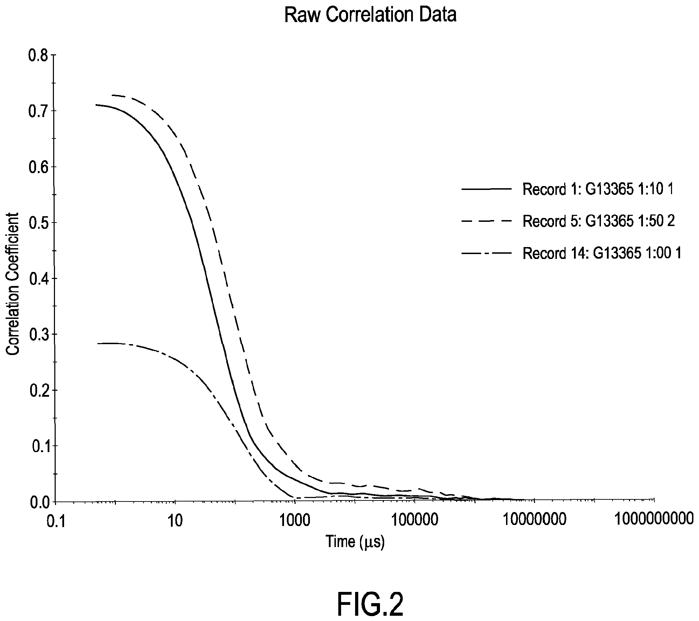

The aggregation phenomena decreased considerably compared to the samples diluted with water, as may be deduced from the analyses of the autocorrelation functions, decidedly more intense and characterised by less noise than those obtained previously. Figure 2 shows the autocorrelation curve of A as an example.

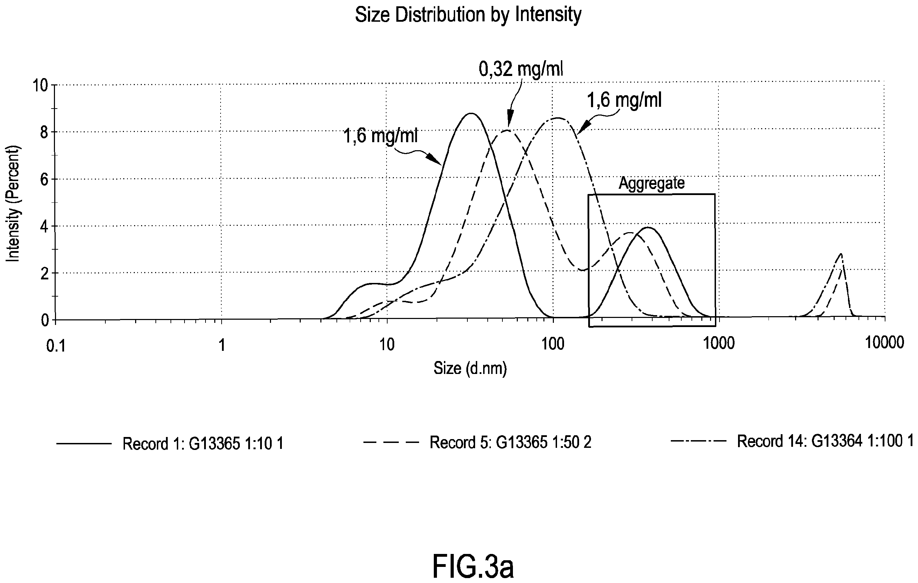

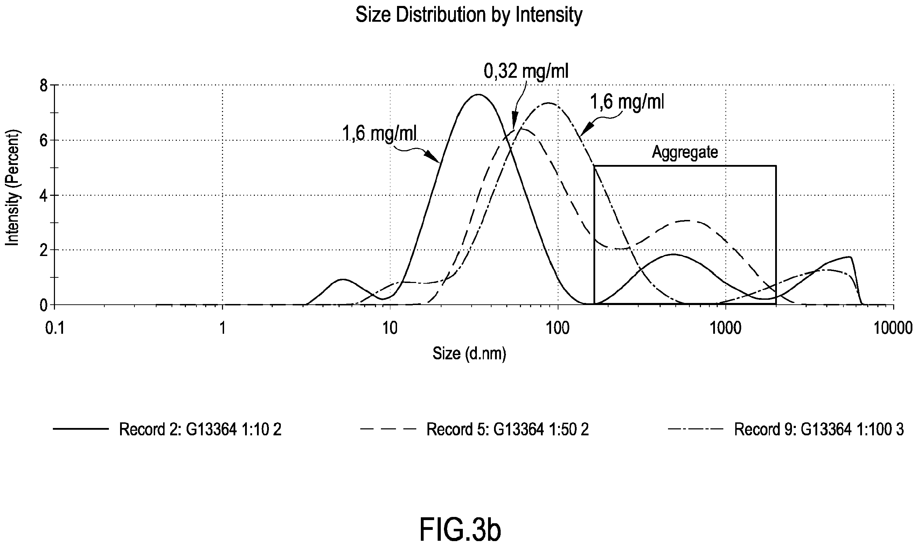

A distribution of the hydrodynamic radii was obtained by processing the correlation curves shown in figures 3a and 3b. As in the case of the samples diluted in water, an increase in the hydrodynamic radii is observed with decreases in concentration, a typical behaviour for charged polymers, which tend to enlarge their conformation in a solution when they are more diluted because they have more "space". Furthermore, as expected, the aggregated species showed to be less intense compared to the results obtained in the absence of salt.

Table 4 shows the Z-average values obtained, along with the corresponding standard deviations.

**Table 4: PCS results with dilution in 0.1M NaNO$_3$**

| Sample | [HA] (mg/ml) | [MSM] (mg/ml) | Z-Average (nm) | Standard deviation | Dilution |
|---|---|---|---|---|---|
| **A** | 1.6 | 0 | 20 | 3.4 | NaNO$_3$ 0.1M |
|  | 0.32 | 0 | 39 | 4.1 |  |
|  | 0.16 | 0 | 43 | 8.1 |  |
| **B** | 1.6 | 5.0 | 19 | 0.5 | NaNO$_3$ 0.1M |
|  | 0.32 | 1.0 | 37 | 2.1 |  |
|  | 0.16 | 0.5 | 39 | 6.1 |  |

A further experiment was conducted, diluting the samples with a pH 5.5 acetate buffer solution so as to have the carboxyl of the hyaluronic acid partially acidified. Table 5 shows the Z-average values obtained.

**Table 5: PCS results with dilution in pH 5.5 acetate buffer**

| Sample | [HA] (mg/ml) | [MSM] (mg/ml) | Z-Average (nm) | Standard deviation | Dilution |
|---|---|---|---|---|---|
| **A** | 0.32 | 0 | 30 | 1.58 | Acetate buffer |
|  | 0.16 | 0 | 38 | 2.68 |  |
| **B** | 0.32 | 1.0 | 31 | 3.32 | Acetate buffer |
|  | 0.16 | 0.5 | 47 | 2.05 |  |

The correlation functions are good for both samples and under both concentration conditions, and the presence of aggregates is decidedly reduced. Comparing the results obtained, the sample containing HA + MSM (**B**) shows a significantly higher Z-Average value than hyaluronic acid as such, and this is in accordance with the GPC-TDA results (Table 2).

A further investigation was conducted, measuring the surface charge, zeta potential (Zp), which enables an evaluation of the goodness of dispersion of the particles in the solution and aggregation phenomena. In fact, if the Zp value is greater than about +30mV or less than -30mV, the particles can be considered to be well dispersed in the solvent; however, if the value tends toward values close to zero, it means that the surface charges are cancelled out due to phenomena of electrostatic interaction and thus the molecules aggregate. This parameter is useful in the case of charged polymers because it provides an idea of the variation in electrostatic interactions when two molecules are mixed. The zeta potential is normally measured by dispersing the polymer in water in such a way as not to decrease the surface charges with a polymer-solvent interaction. In this case the dilution of the samples is carried out with the acetate buffer, as it was seen from the size analyses that, in the presence of water alone, the sample aggregates a great deal and therefore the values of potential would be unstable and scarcely reliable. It was thus preferred to mask the surface charges to a minimum extent and simultaneously reduce aggregation phenomena with the acetate buffer also used for the size analyses.

Table 6 shows the results obtained under the different experimental conditions.

**Table 6: Zp results**

| Sample | Zp (mV) pH 5.5 acetate buffer | |
|---|---|---|
| [HA] (mg/ml) | 0.32 | 0.16 |
| A | -15 ± 2.12 | -15 ± 1.55 |
| B | -8 ± 0.71 | -11 ± 2.15 |

A decrease in Zp is observed in sample B versus sample A, indicating that the presence of MSM reduces the negative charge of the hyaluronic acid, hence by means of an electrostatic interaction.

**NMR analysis**

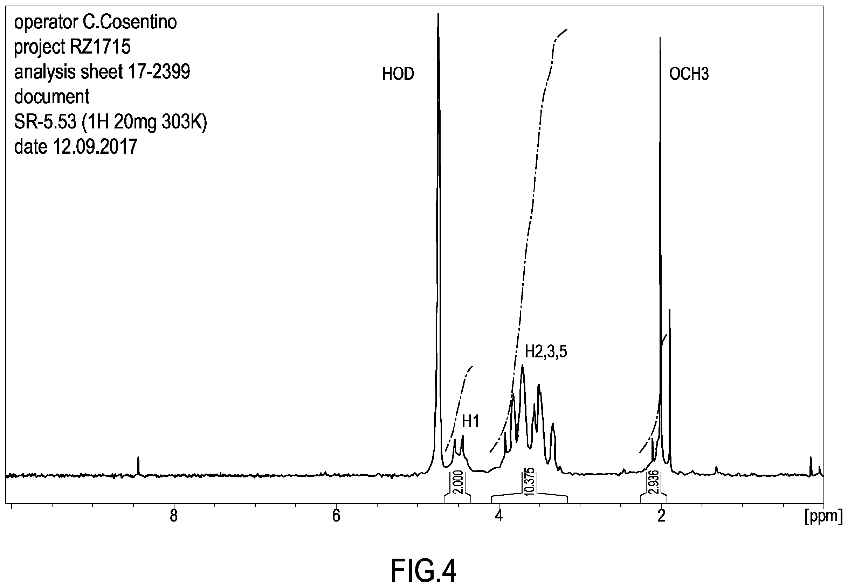

First of all, the proton spectra of the three products were recorded in order to identify the characteristic chemical shifts of the different functional groups and fine tune the acquisition conditions. Figure 4 shows the $^1$H NMR spectrum of HA, sample A.

Two peaks may be observed, relating to two anomeric protons of the disaccharide repeating unit around 4.5 ppm, the signals of the protons in position 2,3,3 of the saccharide ring and, finally, the methyl signal at about 2 ppm relating to N-acetyl glucosamine. The values of the integrals are in accordance with the chemical structure of the polysaccharide, indicating that the parameters used for spectrum acquisition are suitable in order to have a quantitative response.

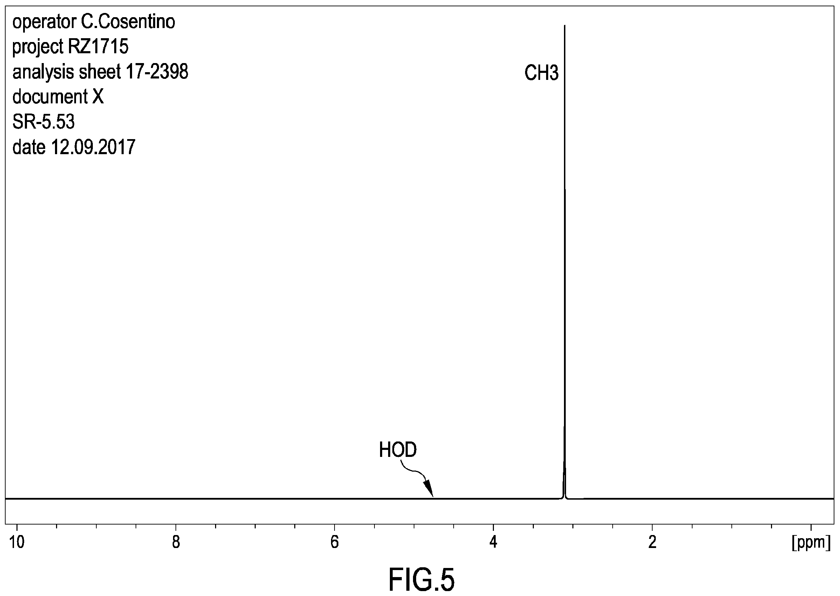

In the next part of the study, the proton spectrum of the MSM sample was recorded; figure 5 shows the NMR profile.

As expected, there is only a signal at about 3 ppm attributable to the two $CH_3$ groups present in the molecule.

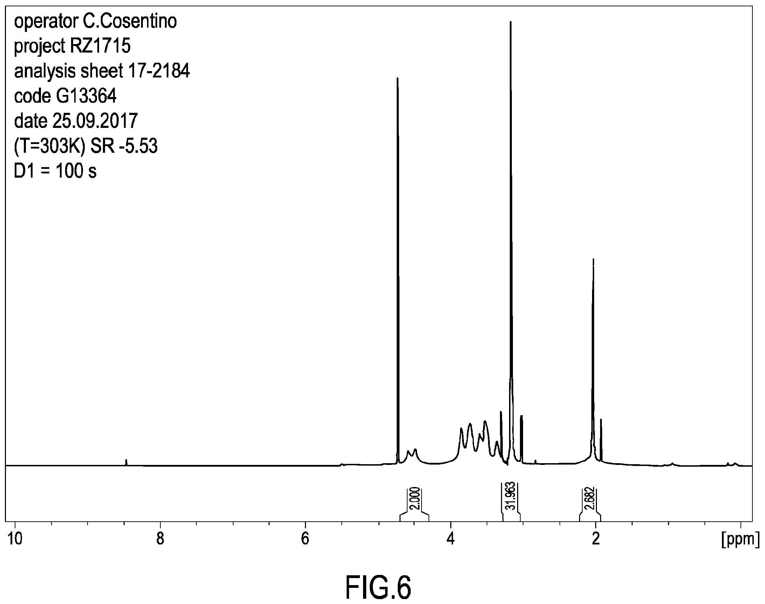

Finally, the $^1$H NMR spectrum of the sample containing HA and MSM according to the present invention was recorded (figure 6):

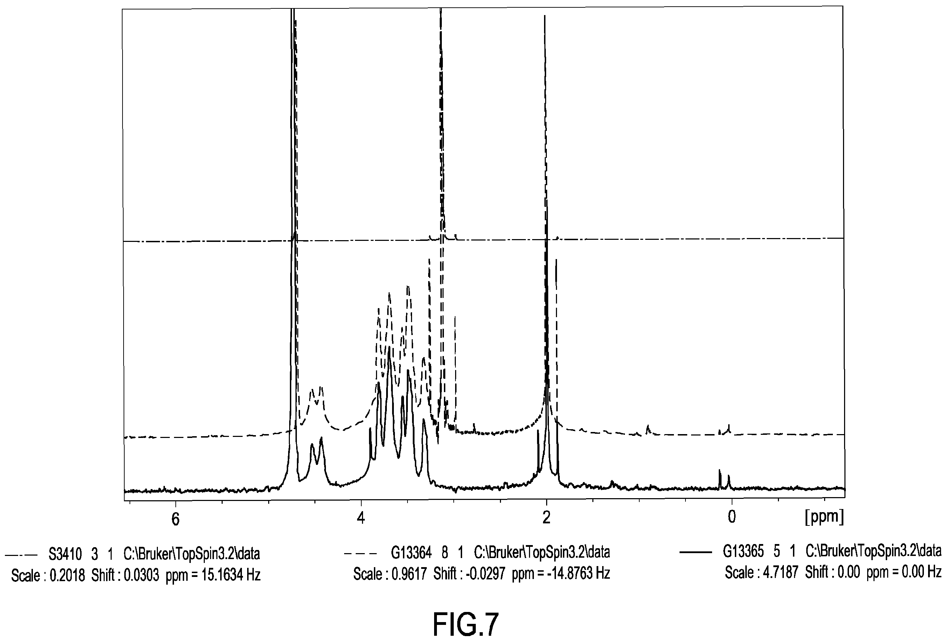

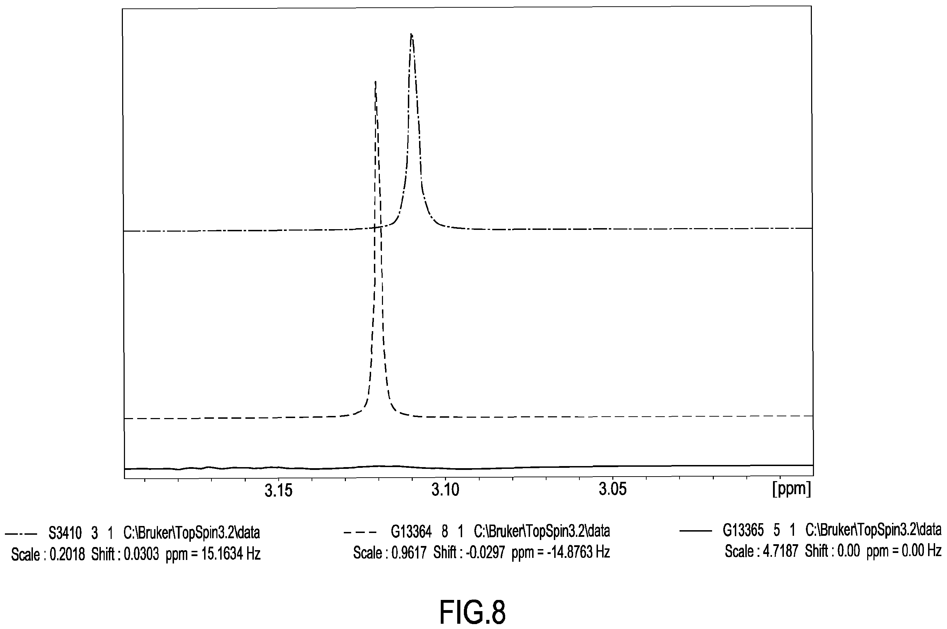

The overlay of the proton spectra of the three samples (Figure 7) shows that the HA signals resonate at the same chemical shift irrespective of the presence of MSM in the solution, whereas the signal related to the methyls of the organic molecule shifts once in a mixture with the polysaccharide, as highlighted by the expansion presented in figure 8.

This shift might depend on an interaction between the hyaluronic acid and MSM that leads to a change in the chemical shift.

In order to confirm an interaction between the two structures, NMR DOSY (Diffusion Ordered SpectroscopY) measurements were performed; this enables the diffusion coefficient to be evaluated from the capacity of a nucleus to change position in the magnetic field. Applying a magnetic field gradient (i.e. a magnetic field that varies progressively along the vertical axis, along the direction of the magnetic field) which is added to the static magnetic field of the instrument, every point will have a magnetic field that also depends on its position; the magnetic field affecting (B) will be expressed by:

$$B = B_0 + G_z z$$

Where

Bo is the static magnetic field,
Gz is the coefficient of the gradient,
z is the position along the vertical axis.

Therefore, two atoms of hydrogen that have the same chemical neighbourhood in the absence of a gradient, but a different position along the vertical, will give an absorption signal at the same frequency, whereas in the presence of a gradient they will give rise to two distinct lines. With a combination of a radiofrequency pulse and calibrated time intervals it is possible to bring the two lines to the same value, that is, to refocus them (as if we were to put a double image into focus) so as to again obtain a single line. This takes place in the assumption that during the pulse time the hydrogen atoms (or rather, the molecules that contain them) have not moved. If, by contrast, they have moved (as a result of the fact that they are diffused, that is, they move in the solution), a part of the signal will be lost because it will not be "refocused" and will get lost in the background noise. Therefore, in the DOSY experiment the intensity of the resonance signals of the hydrogen nuclei (I) depends on the diffusion coefficient D, which measures the speed at which the molecules move, and the time interval $\tau$ used in the 'refocusing'.

$$I = I_0\, e^{-bD}$$

Where $I_0$ is the initial intensity, b is a coefficient that depends on the time $\tau$ and D is the diffusion coefficient.

The diffusion coefficient is in turn directly proportional to the temperature T and inversely proportional to the hydrodynamic radius (Rh), i.e. the size of the molecule:

$$D = \text{const } T/Rh$$

By carrying out a series of experiments with different times (i.e. varying the coefficient b), it is possible to determine the diffusion coefficient D for each signal.

If we analyse a mixture of substances, in the NMR spectrum we will see all the signals of the hydrogen nuclei of the molecules present. With the DOSY experiment, the signals can be separated based on their diffusion coefficient. The signals of the nuclei of the same molecule will give the same value of D, whilst those of different molecules with a different D will be distinct. This distinction is highlighted by dividing the signals in a second dimension that distinguishes the values of the logarithm of D. The hydrogen nucleus signals of the same molecule will be on the same line and the signals of different molecules will be on different lines.

Therefore, with this experiment it is possible to separate the spectra of individual molecules without separating the respective compounds, but rather based on their capacity to migrate; it is thus said to be the NMR equivalent of a chromatograph such as HPLC or GC. Table 7 shows the results obtained from the DOSY experiments carried out on three samples:

**Table 7: Results of DOSY experiments**

| Sample | $CH_3$ $(m^2/s)$ | OAc $(m^2/s)$ | H1 $(m^2/s)$ |
|--------|------------------|---------------|--------------|
| S3410  | $1.27 \times 10^{-9}$ | - | - |
| A      | - | $5.74 \times 10^{-12}$ | $12.9 \times 10^{-12}$; $7.91 \times 10^{-12}$ |
| B      | $1.22 \times 10^{-9}$ | $1.05 \times 10^{-12}$ | $2.99 \times 10^{-12}$; $2.21 \times 10^{-12}$ |

It may be observed that the diffusion coefficients both of the methyls of MSM and the protons of the polysaccharide are lower in sample B than in the single components, A and S3410. This confirms an interaction between the two molecules which leads to a decrease in their mobility in the solution, with a reduction, therefore, of the diffusion coefficient and an associated increase in the hydrodynamic radius, these parameters being inversely proportional.

**Conclusions**

The comparative study conducted on the solution containing HA and the one of HA with MSM in a weight ratio of 1:3 (the latter according to the invention) provided physicochemical parameters useful for characterising not only the polymeric component, but also the interaction between HA and MSM.

Firstly, an increase was observed in the average molecular weights, the hydrodynamic radius and the intrinsic viscosity of the sample containing HA + MSM according to the invention compared to HA as such. The increase in size was also confirmed by the dynamic light scattering results, although the test conditions were necessarily different. Furthermore, the zeta potential measurements revealed a change in the surface charge of hyaluronic acid. In particular, a decrease in the zeta potential was observed in the presence of MSM.

Finally, the interaction between HA and MSM was confirmed by NMR spectroscopy, based both on the change observed in the chemical shift of the small organic molecule and the measurement of the diffusion coefficients of the protons involved in the HA-MSM binary system.

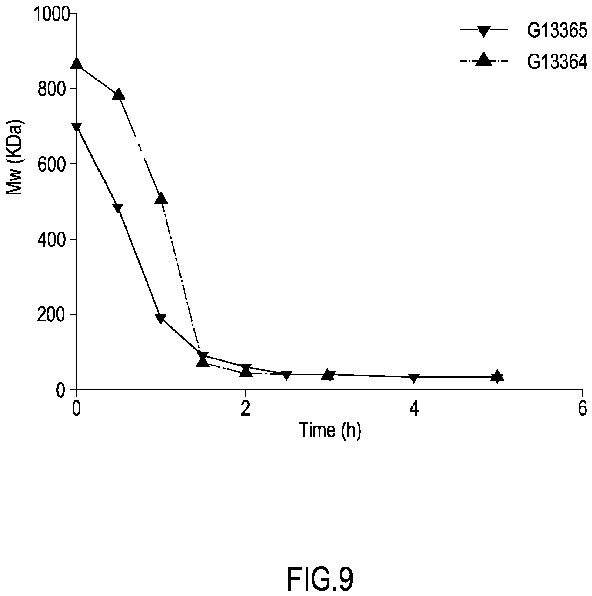

Figure 9 shows the hydrolysis curves of product A (G13365) containing HA alone and product B (G13364) containing HA + MSM (all the experiments were conducted in duplicate).

The different kinetics were followed with a GPC-TDA analysis of the different samples.

It was shown that the molecular weights at the end of hydrolysis are analogous in the two products, but the speed at which a significant decrease in molecular weight is reached changes, i.e. decidedly slower hydrolysis kinetics are observed for product B according to the invention.

EXPERIMENTAL PART (B)

Comparative assessment in vitro of the potential anti-inflammatory activity of a composition of the invention.

**I) Aim of the test**

The aim of the test is to assess whether the tested products (composition of the invention, comparative compounds and controls), at different concentrations, are capable of reducing the production of cytokines in vitro. For this reason, their effectiveness in reducing the synthesis of interleukin-1$\beta$ (IL-1$\beta$), interleukin-6 (IL-6) and tumor necrosis factor $\alpha$ (TNF-$\alpha$) was tested. It is deemed that such an ability makes the product a potential soothing or anti-inflammatory candidate in vivo, capable of reducing the process of skin reddening, irritation and inflammation.

The skin is the largest organ of the human body, with a unique structure making it a barrier against infection and other potential environmental risks/hazards. Skin irritation represents a local, reversible non-immunological reaction of an inflammatory type, characterised by erythema and oedema.

One way to establish the soothing or anti-inflammatory action of a cosmetic or pharmaceutical product is to measure the inflammatory markers indicative of skin irritation.

Various irritating substances can give rise to inflammatory processes. The main mechanism used by epidermal cells to participate in inflammatory reactions of the skin is the production of and response to cytokines. In the epidermis keratinocytes are the main source of cytokines together with Langerhans cells, skin mast cells, dendritic cells and macrophages. While keratinocytes at rest produce some cytokines constitutionally, numerous environmental stimuli, including ultraviolet radiation and chemical agents, can stimulate them to release inflammatory cytokines (IL-1, TNF-a), chemotactic cytokines (such as IL-8) and cytokines that promote cell proliferation (such as IL-6 and IL-7).

IL-1$\beta$ is a member of the interleukin 1 family. This cytokine is produced by macrophages activated as proprotein, which is converted into its active form through proteolytic cleavage by caspase 1. This cytokine is an important mediator of the inflammatory response and is involved in numerous cellular activities, including proliferation, cell differentiation and apoptosis. An increase in the production of IL-1$\beta$ causes a series of different autoinflammatory syndromes.

TNF-$\alpha$ is a cytokine that binds to the receptors TNFR-55 and TNFR-75, which are expressed on all somatic cells. It is produced by different types of cells, but above all by activated monocytes and it can induce cell death in several lines of tumour cells. TNF-$\alpha$ is a potent pyrogen, it triggers the cascade of cytokines and increases vascular permeability, thus recruiting macrophages and neutrophils at the sites of bacterial, fungal, viral or parasitic infection. TNF-$\alpha$ plays a role in disorders of an inflammatory origin.

**II) Material**

II.1) Cell lines and culture conditions:

The test was conducted on human keratinocytes (Huker) cultured in DMEM (Dulbecco's Modified Eagle's Medium) supplemented with foetal bovine serum (10%) and glucose (4.5 g/l) and incubated under standard culture conditions (37°C, 5% CO2). Best practices for cell culture were applied.

II.2) Tested samples:

A. Joyflex 2% (comparative compound): Hyaluronic Acid sodium salt 2% (Mw HA 800-1200 kDa).
B. Joyflex Proakjn (compound according to the invention): Hyaluronic Acid sodium salt 1.6% (Mw HA 800-1200 kDa) +MSM 5%.
C. Hymovis (comparative compound): Hyaluronic Acid sodium salt hexadecylamide (Mw HA 800 kDa).
D. Synolis (comparative compound): Hyaluronic Acid sodium salt 1.6% (Mw HA 2200 kDa) + sorbitol 4%.

Caption, HA: hyaluronic acid

**III) Methods**

III.1) Assessment of cell viability

Prior to the cytokine assay, a cell viability assay was conducted with the aim of choosing the concentrations on which to carry out the subsequent assay. At the end of the assay the viability test was repeated (in the absence and in the presence of LPS) to establish whether the treatments performed had a negative effect on the cells. Cell viability was assessed by means of the MTT test; MTT [3-(4,5-dimethylthiazol-2-yl)-2,5- diphenyltetrazolium bromide] is a yellow tetrazole compound that is reduced by cells to purple formazan. This conversion is caused by the NADPH or NADH produced by dehydrogenases present in metabolically active cells. The cells were treated with MTT (1 mg/ml) and incubated for 3 hours under standard conditions. At the end of this period the MTT solution was eliminated and 100 $\mu$l of isopropanol were added in each well to dissolve the formazan crystals that had formed. Absorbance (optical density, OD) was determined by means of a spectrophotometric reading at a wavelength of 540 nm.

III.2) Cytokine assay

The test was conducted in parallel on two series of cells: an untreated one and one treated with lipopolysaccharide (LPS) to stimulate the production of interleukins. The cells were treated with preestablished concentrations of the product to be tested and the positive control (CQ). Cells not treated with the sample represent the negative control. The content of IL-1$\beta$, IL-6 and TNF-$\alpha$ in the culture medium was determined by ELISA (Enzyme-Linked Immunosorbent Assay), a very widely used biochemical technique for quantifying proteins and peptides. In an ELISA a specific antigen for the interleukin to be identified is immobilised on a solid surface (the bottom of a well), to which the assay medium is added. Detection is achieved by means of a biotinylated secondary antibody made to react with streptavidin-HRP. The colorimetric reaction is proportional to the amount of cytokine present. The results were read with a spectrophotometer at 450 nm. The values obtained were then interpolated in a standard curve for interleukin.

**IV) Results**

IV.1) Assessment of cell viability

Table 8:

| | Sample concentration (mg/ml) | | | | | | | |
|---|---|---|---|---|---|---|---|---|
| | **0.03 91** | **0.0781** | **0.156** | **0.313** | **0.625** | **1.25** | **2.5** | **5.0** |
| **A** | 98.3 8 | 108.64 | 103.78 | 113. 91 | 105. 62 | 114. 99 | 103. 19 | 88.50 |
| **B** | 105. 84 | 117.35 | 116.19 | 113. 02 | 105. 25 | 116. 53 | 120. 01 | 100.2 9 |
| **C** | 101. 00 | 108.64 | 105.96 | 110. 59 | 102. 41 | 108. 34 | 104. 13 | 108.1 2 |
| **D** | 104. 78 | 99.60 | 99.18 | 113. 08 | 108. 11 | 113. 68 | 115. 16 | 105.6 2 |

The optical density (OD) measured at 540 nm is proportional to cell viability. The percentages were calculated on the basis of the absorbance values at 540 nm, considering the absorbance of the negative control (untreated cells) as 100%. Sample A: Joyflex 2%; Sample B: Joyflex Proakjn; Sample C: Hymovis; Sample D: Synolis

The keratinocytes were treated with scalar concentrations of the tested products (1:2 dilutions starting from 5.0 mg/ml) and it emerged that cell viability does not decrease significantly at the tested concentrations (Table 8); based on the results obtained, concentrations of 5.0, 2.0 and 1.0 mg/ml were chosen to carry out the cytokine assay.

IV.2) Cytokine assay

For the cytokine assay the cells were treated with the test sample at the established concentrations. Untreated cells represent the negative control. A substance with known anti-inflammatory activity was used as a positive control (CQ). Cytokine production was induced using lipopolysaccharide (LPS). At the end of the assay a second MTT test was performed to verify cell viability.

IV.2.1) Test on cell viability in the absence and presence of LPS

Table 9

| | LPS (-) | | | LPS (+) | | |
|---|---|---|---|---|---|---|
| | Sample (mg/ml) | | | Sample (mg/ml) | | |
| | **5.0** | **2.0** | **1.0** | **5.0** | **2.0** | **1.0** |
| **A** | 99.11 | 97.87 | 97.95 | 86.2 2 | 88.40 | 89.2 4 |
| **B** | 95.67 | 94.70 | 95.98 | 87.5 7 | 85.02 | 84.8 3 |
| **C** | 92.77 | 91.64 | 94.24 | 82.2 2 | 82.75 | 81.4 0 |
| **D** | 84.22 | 88.94 | 86.11 | 85.0 2 | 82.79 | 81.4 9 |

The absorbance measured at 540 nm is proportional to cell viability. The percentages were calculated on the basis of the absorbance values at 540 nm, considering the absorbance of the negative control (untreated cells) as 100%. Sample A: Joyflex 2%; Sample B: Joyflex Proakjn; Sample C: Hymovis; Sample D: Synolis
The treatment with LPS and with the tested sample did not bring about any significant variations in cell viability (Table 9).

IV.2.2) IL-1β Assay

Table 10

| | IL-1β (pg/ml) | | | | | Reduction (%) | | | | |
|---|---|---|---|---|---|---|---|---|---|---|
| | Sample (mg/ml) | | | Positive | C Q | Sample (mg/ml) | | | Positive | CQ |
| | **5.0** | **2.0** | **1.0** | | | **5.0** | **2.0** | **1.0** | | |
| **A** | 206.0 5 | 204.47 | 205.26 | | | 11.09 | 11.7 7 | 11.4 3 | | |
| **B** | 186.6 2 | 195.21 | 197.89 | 231.75 | 131.79 | 19.47 | 15.7 6 | 14.6 1 | 0.00 | 43.13 |
| **C** | 201.6 2 | 202.93 | 208.81 | | | 13.00 | 12.4 3 | 9.90 | | |
| **D** | 207.1 0 | 201.44 | 202.85 | | | 10.64 | 13.0 8 | 12.4 7 | | |

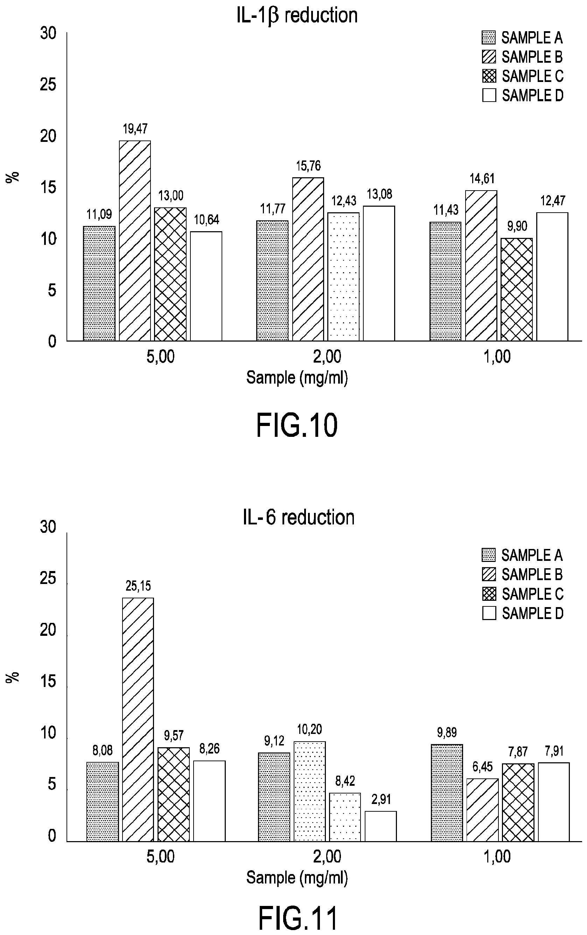

The absorbance measured at 450 nm is directly proportional to the amount of IL-1β produced by cells. The values were interpolated with a standard curve for IL-1β and the results expressed as pg/ml. Sample A: Joyflex 2%; Sample B: Joyflex Proakjn; Sample C: Hymovis; Sample D: Synolis
Sample B showed to be capable of reducing the production of IL-1β at all of the tested concentrations (Table 10 and Figure 10). In the presence of samples A, C and D, the reduction of IL-1β is significantly lower compared to sample B.

IV.2.3) IL-6 Assay

Table 11

| | | IL-6 (pg/ml) | | | | | Reduction (%) | | | | |
|---|---|---|---|---|---|---|---|---|---|---|---|
| | | Sample (mg/ml) | | | Positive | C Q | Sample (mg/ml) | | | Positive | CQ |
| | | 5.0 | 2.0 | 1.0 | | | 5.0 | 2.0 | 1.0 | | |
| | A | 981.4 1 | 970.30 | 962.15 | 1067.70 | 460.30 | 8.08 | 9.12 | 9.89 | 0.00 | 56.89 |
| | B | 799.1 9 | 958.81 | 998.81 | | | 25.15 | 10.20 | 6.45 | | |
| | C | 965.4 8 | 1016.2 2 | 983.63 | | | 9.57 | 4.82 | 7.87 | | |
| | D | 979.5 6 | 1036.5 9 | 983.26 | | | 8.26 | 2.91 | 7.91 | | |

The absorbance measured at 450 nm is directly proportional to the amount of IL-6 produced by cells. The values were interpolated with a standard curve for IL-6 and the results expressed as pg/ml. Sample A: Joyflex 2%; Sample B: Joyflex Proakjn; Sample C: Hymovis; Sample D: Synolis

Sample B showed to be capable of reducing the production of IL-6 at the tested concentrations of 5.0 and 2.0 mg/ml (Table 11 and Figure 11). At the concentration of 5.0 mg/ml, sample B is much more effective than the other samples A, C and D in reducing the levels of IL-6.

IV.2.4) TNF-$\alpha$ Assay

Table 12

| | | TNF-$\alpha$ (pg/ml) | | | | | Reduction (%) | | | | |
|---|---|---|---|---|---|---|---|---|---|---|---|
| | | Sample (mg/ml) | | | Positive | C Q | Sample (mg/ml) | | | Positive | CQ |
| | | 5.0 | 2.0 | 1.0 | | | 5.0 | 2.0 | 1.0 | | |
| | A | 519.9 2 | 517.42 | 505.75 | 560.75 | 264.08 | 7.28 | 7.73 | 9.81 | 0.00 | 52.91 |
| | B | 443.2 5 | 489.92 | 499.08 | | | 20.95 | 12.63 | 11.0 0 | | |
| | C | 512.4 2 | 519.92 | 510.75 | | | 8.62 | 7.28 | 8.92 | | |
| | D | 517.4 2 | 524.08 | 529.92 | | | 7.73 | 6.54 | 5.50 | | |

The absorbance measured at 450 nm is directly proportional to the amount of TNF-$\alpha$ produced by cells. The values were interpolated with a standard curve for TNF-$\alpha$ and the results expressed as pg/ml. Sample A: Joyflex 2%; Sample B: Joyflex Proakjn; Sample C: Hymovis; Sample D: Synolis

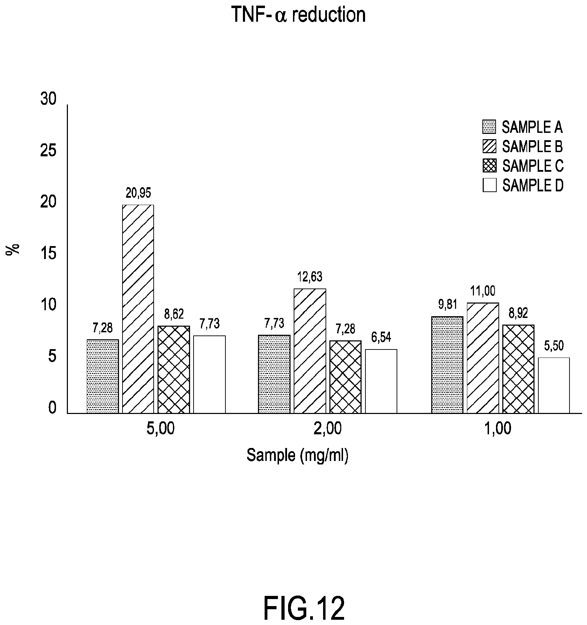

Sample B showed to be capable of reducing the production of TNF-$\alpha$ at all the tested concentrations (Table 12 and Figure 12).

At the concentrations of 5.0 and 2.0 mg/ml, sample B is more effective than the other samples in reducing the levels of TNF-$\alpha$.

VI.3) Summary

From the results obtained it emerged that sample B (Joyflex Proakjn, compound according to the invention) is capable of reducing cytokine levels in vitro in cultures of keratinocytes. In particular, it reduces the levels of IL-1$\beta$ and TNF-$\alpha$ at all of the tested concentrations (5.0, 2.0 and 1.0 mg/ml) and reduces the levels of IL-6 at the tested concentrations of 5.0 and 2.0 mg/ml. Sample B is more effective than the other samples A, C and D (comparative compounds) in reducing the cytokines considered (IL-1$\beta$, IL-6 and TNF-a); the effect is particularly evident at the tested concentration of 5.0 mg/ml.

**Claims**

**1.** A liquid composition comprising (i) an effective amount of a mixture that comprises or, alternatively, consists of at least (a) hyaluronic acid, or a salt thereof, and (b) methylsulfonylmethane (MSM), wherein (a) and (b) are in a ratio by weight between 1:2 and 1:4, and (ii) a physiologically acceptable carrier; wherein said composition is for use in a method for the preventive or therapeutic treatment of at least one disorder or pathology deriving from an oxidative and/or inflammatory state, wherein said disorder or pathology is at least one selected from: vulvovaginal atrophy, vaginal dryness, abacterial cystitis, prostatitis, rhinitis, sinusitis, rhinorrhoea, allergic rhinitis, haemorrhoids, anal fissures, rhagades and lesions of the rectal canal;
wherein said composition is administered to a subject in a state of need and wherein said composition is administered to said subject topically.

2. The composition for use according to claim 1, wherein the ratio by weight between (a) and (b) is between 1:2.5 and 1:3.5, preferably wherein said ratio is 1:3.

3. The composition for use according to one of the preceding claims, wherein the hyaluronic acid, or a salt thereof, (a) has a weight average molecular weight of 700 KDa to 1400 KDa, preferably 800 to 1200 KDa.

4. The composition for use according to any one of the preceding claims, wherein the hyaluronic acid, or a salt thereof, (a) is present in an amount of 0.5% to 6%, more preferably 1% to 3.5% or 1.5% to 3.2% or 4.5% to 5.5% relative to the total weight of the composition and/or MSM (b) in an amount of 1% to 12%, more preferably 2% to 7% or 3% to 6.8% or 9% to 11%, relative to the total weight of the composition.

5. The composition for use according to any one of the preceding claims, wherein said mixture comprises, or alternatively, consists of a non-covalent complex between hyaluronic acid (a) and MSM (b).

6. The composition for use according to claims 1-5, wherein said composition is administered to said subject topically and said composition is formulated in liquid form.

7. The composition for use according to claims 1-5, wherein said composition is administered to said subject topically and said composition is formulated in gel form.

8. The composition for use according to any one of claims 1 to 7, wherein said composition is administered to said subject rectally.

**Patentansprüche**

1. Flüssige Zusammensetzung umfassend (i) eine wirksame Menge einer Mischung umfassend oder alternativ bestehend aus mindestens (a) Hyaluronsäure oder ein Salz davon und (b) Methylsulfonylmethan (MSM), wobei (a) und (b) in einem Gewichtsverhältnis zwischen 1:2 und 1:4 vorliegen, und (ii) einen physiologisch akzeptablen Träger; zur Verwendung in einem Verfahren zur vorbeugenden oder therapeutischen Behandlung von mindestens einer Erkrankung oder Pathologie, die von einem oxidativen und/oder entzündlichen Zustand herrührt, wobei die Erkrankung oder Pathologie mindestens eine ist, ausgewählt aus: Vulvovaginalatrophie, vaginale Trockenheit, abakterielle Zystitis, Prostatitis, Rhinitis, Sinusitis, Rhinorrhoe, allergische Rhinitis, Hämorrhoiden, Analfissuren, Rhagaden und Läsionen des Rektalkanals;
wobei die Zusammensetzung einem Patienten bei Bedarf verabreicht wird und wobei die Zusammensetzung dem Patienten topisch verabreicht wird.

2. Zusammensetzung zur Verwendung gemäß Anspruch 1, wobei das Gewichtsverhältnis zwischen (a) und (b) zwischen 1:2,5 und 1:3,5 liegt, vorzugsweise wobei das Verhältnis 1:3 ist.

3. Zusammensetzung zur Verwendung gemäß mindestens einem der vorhergehenden Ansprüche, wobei die Hyaluronsäure oder ein Salz davon, (a) ein gewichtsmittleres Molekulargewicht von 700 KDa bis 1400 KDa, vorzugsweise 800 bis 1200 KDa, aufweist.

4. Zusammensetzung zur Verwendung gemäß mindestens einem der vorhergehenden Ansprüche, wobei die Hyaluronsäure oder ein Salz davon, (a) in einer Menge von 0,5% bis 6%, noch bevorzugter 1% bis 3,5% oder 1,5% bis 3,2% oder 4,5% bis 5,5%, bezogen auf das Gesamtgewicht der Zusammensetzung, und/oder MSM (b) in einer Menge von 1% bis 12%, noch bevorzugter 2% bis 7% oder 3% bis 6,8% oder 9% bis 11%, bezogen auf das Gesamtgewicht der Zusammensetzung, vorhanden ist.

**5.** Zusammensetzung zur Verwendung gemäß mindestens einem der vorhergehenden Ansprüche, wobei die Mischung einen nicht-kovalenten Komplex zwischen Hyaluronsäure (a) und MSM (b) umfasst oder alternativ daraus besteht.

**6.** Zusammensetzung zur Verwendung gemäß mindestens einem der Ansprüche 1-5, wobei die Zusammensetzung dem Patienten topisch verabreicht wird und die Zusammensetzung in flüssiger Form formuliert ist.

**7.** Zusammensetzung zur Verwendung gemäß mindestens einem der Ansprüche 1-5, wobei die Zusammensetzung dem Patienten topisch verabreicht wird und die Zusammensetzung in Gelform formuliert ist.

**8.** Zusammensetzung zur Verwendung gemäß mindestens einem der Ansprüche 1-7, wobei die Zusammensetzung dem Patienten rektal verabreicht wird.

**Revendications**

**1.** Composition liquide comprenant (i) une quantité efficace d'un mélange qui comprend ou, en variante, est constitué au moins (a) de l'acide hyaluronique ou un sel de celui-ci, et (b) du méthylsulfonylméthane (MSM), dans laquelle (a) et (b) sont en un rapport en poids compris entre 1/2 et 1/4 ; et (ii) un véhicule physiologiquement acceptable ; dans laquelle ladite composition est destinée à être utilisée dans une méthode de traitement prophylactique ou thérapeutique d'au moins un trouble ou pathologie dérivant d'un état oxydatif et/ou inflammatoire, dans laquelle ledit trouble ou pathologie est au moins l'un choisi parmi : une atrophie vulvo-vaginale, une sécheresse vaginale, une cystite abactérienne, une prostatite, une rhinite, une sinusite, une rhinorrhée, une rhinite allergique, des hémorroïdes, des fissures anales, des rhagades et des lésions du canal rectal ; dans laquelle ladite composition est administrée à un sujet en ayant besoin, et dans laquelle ladite composition est administrée audit sujet par voie topique.

**2.** Composition destinée à être utilisée selon la revendication 1, dans laquelle le rapport en poids entre (a) et (b) est compris entre 1/2,5 et 1/3,5, de préférence dans laquelle ledit rapport est de 1/3.

**3.** Composition destinée à être utilisée selon l'une des revendications précédentes, dans laquelle l'acide hyaluronique, ou un sel de celui-ci, (a) a une masse moléculaire moyenne en masse de 700 kDa à 1400 kDa, de préférence de 800 à 1200 kDa.

**4.** Composition destinée à être utilisée selon l'une quelconque des revendications précédentes, dans laquelle l'acide hyaluronique, ou un sel de celui-ci, (a) est présent en une quantité de 0,5 % à 6 %, mieux encore de 1 % à 3,5 % ou de 1,5 % à 3,2 % ou de 4,5 % à 5,5 % par rapport au poids total de la composition et/ou le MSM (b) est présent en une quantité de 1 % à 12 %, mieux encore de 2 % à 7 % ou de 3 % à 6,8 % ou de 9 % à 11 % par rapport au poids total de la composition.

**5.** Composition destinée à être utilisée selon l'une quelconque des revendications précédentes, dans laquelle ledit mélange comprend ou, en variante, est constitué d'un complexe non covalent entre l'acide hyaluronique (a) et le MSM (b).

**6.** Composition destinée à être utilisée selon l'une quelconque des revendications 1 à 5, dans laquelle ladite composition est administrée audit sujet par voie topique et ladite composition est formulée sous forme liquide.

**7.** Composition destinée à être utilisée selon l'une quelconque des revendications 1 à 5, dans laquelle ladite composition est administrée audit sujet par voie topique et ladite composition est formulée sous forme de gel.

**8.** Composition destinée à être utilisée selon l'une quelconque des revendications 1 à 7, dans laquelle ladite composition est administrée audit sujet par voie rectale.

Raw Correlation Data

FIG.1a

FIG.1b

Raw Correlation Data

FIG.2

Size Distribution by Intensity

FIG.3a

Size Distribution by Intensity

FIG.3b

EP 3 697 444 B1

FIG.4

operator C.Cosentino
project RZ1715
analysis sheet 17-2398
document X
SR-5.53
date 12.09.2017

CH3

HOD

FIG.5

EP 3 697 444 B1

EP 3 697 444 B1

operator C.Cosentino
project RZ1715
analysis sheet 17-2184
code G13364
date 25.09.2017
(T=303K) SR -5.53
D1 = 100 s

2.000    31.963    2.682

10    8    6    4    2    [ppm]

FIG.6

—·— S3410  3  1  C:\Bruker\TopSpin3.2\data
Scale : 0.2018  Shift : 0.0303  ppm = 15.1634 Hz

— — — G13364  8  1  C:\Bruker\TopSpin3.2\data
Scale : 0.9617  Shift : -0.0297  ppm = -14.8763 Hz

——— G13365  5  1  C:\Bruker\TopSpin3.2\data
Scale : 4.7187  Shift : 0.00  ppm = 0.00 Hz

FIG.7

| ─·─ S3410  3  1  C:\Bruker\TopSpin3.2\data | ─ ─ ─ G13364  8  1  C:\Bruker\TopSpin3.2\data | ──── G13365  5  1  C:\Bruker\TopSpin3.2\data |
| Scale : 0.2018  Shift : 0.0303 ppm = 15.1634 Hz | Scale : 0.9617  Shift : -0.0297 ppm = -14.8763 Hz | Scale : 4.7187  Shift : 0.00 ppm = 0.00 Hz |

FIG.8

FIG.9

FIG.10

FIG.11

FIG.12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017025903 A1
- EP 1444984 A1

- WO 9405293 A1
- US 2015094279 A1

**Non-patent literature cited in the description**

- *CHEMICAL ABSTRACTS,* 67-71-0

- Compendium of Polymer Terminology and Nomenclature IUPAC Recommendations 2008. Purple book. RSC Publishing